# EUROPEAN PATENT SPECIFICATION

(11) **EP 3 558 185 B1**
(45) Date of publication and mention of the grant of the patent: **29.11.2023**
(21) Application number: 17882478.5
(22) Date of filing: 22.12.2017
(51) Int. Cl.: A61F 13/00, A61F 13/02, A61L 15/16, A61M 1/00, A61M 3/02, A61M 27/00

(54) **DEVICES FOR WOUND TREATMENT**
VORRICHTUNGEN ZUR WUNDBEHANDLUNG
DISPOSITIFS POUR LE TRAITEMENT DES PLAIES

(30) Priority: 22.12.2016 US 201662438257 P
(43) Date of publication of application: 30.10.2019
(73) Proprietor: Applied Tissue Technologies LLC, Hingham, MA 02043 (US)
(72) Inventor: ERIKSSON, Elof, Hingham, MA 02043 (US); BROOMHEAD, Michael, Hingham, MA 02043 (US)
(74) Representative: J A Kemp LLP
(86) International application number: PCT/US2017/068310
(87) International publication number: WO 2018/119442

(56) References cited:
- WO-A1-2013/175309
- US-A1- 2009 012 441
- US-A1- 2012 010 578
- US-A1- 2015 119 857
- US-A1- 2015 119 857

## Description

### FIELD OF THE TECHNOLOGY

The disclosure relates generally to wound treatment and, more particularly, to devices and methods for treating wounds with negative pressure and/or therapeutic agents.

### BACKGROUND

Various techniques are employed to treat open wounds. In some cases, open wounds may be treated with moist or dry gauze. However, such treatment may result in excessive pain, dehydration of the wound, loss of fluids and proteins, loss of heat or delayed healing. To delay the appearance of infection, burn wounds may be additionally treated with antibacterial creams and the like.

Wound chambers for protecting open wounds and providing environmental control of the treatment site have been developed. For example, exemplary wound chambers and methods for use are described in U.S. Pat. No. 5,152,757, entitled "System for Diagnosis and Treatment of Wounds," by Elof Eriksson, and U.S. Patent Application Serial No. 11/130,490, entitled "Wound Chamber With Remote Access Portal," by Eriksson et al.

A wound chamber typically includes a chamber for enclosing a predetermined surface area about a wound on a patient. The wound chamber is sealed to the skin immediately adjacent to the wound. However, certain wounds on and around a limb may not be treatable by a wound chamber that is intended for use on relatively flat skin surfaces. Instead, it may be necessary to enclose all or a portion of a limb in a chamber in order to create a chamber environment around the wound. In addition to other features, the wound chamber may have a portal for introducing treatment fluid and treatment additives into the wound chamber and extracting wound fluid and/or air from the wound chamber.

Many wounds can be treated by the application of negative pressure. The method of such treatment has been practiced for many years. The benefits of such treatment can include: reduction of edema; reduction of wound exudate; reduction of wound size; and stimulation of formation of granulation tissue. Existing devices and appliances for the provision of negative pressure wound therapy are complex. Such devices typically encompass a porous insert such as foam or gauze that is placed into the wound; a tube connecting the inner space to a source of suction; a flexible cover draped over these components and sealed to the skin around the wound; an electrically powered suction pump; controls to operate the pump and monitor the system; containers to collect wound fluids; filters to process the materials removed from the wound; and safety systems to prevent harm to the patient and to block the escape of biological materials into the outside environment. These devices are expensive, labor intensive, and restrictive of patient mobility. These devices are generally not considered suitable for wounds on certain areas of the body, including wounds on the face, neck, and head. The many components, particularly the seals around the insert and the tube, tend to leak. Therefore, suction must be applied either continuously or frequently.

Continuous suction is typically achieved by a vacuum pump powered by an electric motor. Such systems require complex means to measure, monitor, and control the operation of the pump to ensure the safety of the patient. In addition, many negative pressure devices are contraindicated in the presence of necrotic tissue, invasive infection, active bleeding, and exposed blood vessels. They require the use of a porous insert (sponge, foam, gauze, mesh, etc.) in the wound. The insert may present two problems: growth of tissue into the insert, and the harboring of infectious and/or undesirable materials in the insert. Wound tissue can grow into and around such inserts, thereby causing adverse results to the healing process. Moreover, such inserts can retain wound fluid and microorganisms, and therefore can become contaminated and/or infected, presenting an adverse effect to the healing process. In addition, the high cost of these devices may deter or delay their use on patients.

Existing negative pressure treatment devices are labor intensive since they require the user to assemble, fit, and customize a number of components. First, the user must prepare, trim, and size an insert of foam, gauze, mesh, or other material that will be placed in the wound. Next, the user must position a tube in the insert, and then cover the tube and insert with a material that is intended to create a leakproof seal. In practice, and as mentioned above, such compositions tend to leak, requiring the frequent application of suction in order to establish and re-establish negative pressure within the space about the wound. In addition, currently available negative pressure devices and systems block the view of the wound, making monitoring and diagnosis more difficult. This is particularly problematic for wounds on the head, neck and face, such that existing negative pressure devices are not suitable for such wound treatment.

US 2015/0119857 A1 discloses a wound treatment device employing negative pressure. WO 2013/175309 A1 discloses devices and methods for treating and closing wounds with negative pressure. US 2009/0012441 A1 discloses a subatmospheric pressure wound therapy dressing.

In accordance with one or more embodiments, devices and methods for treating wounds with negative pressure and/or therapeutic agents are disclosed.

In accordance with one or more aspects, a kit for wound treatment may comprise a wound treatment device comprising a chamber that includes an inner surface and a sealing portion that defines an isolated treatment space, and a plurality of embossed structures arranged in a pattern on the inner surface of the chamber, the structures configured to directly contact a wound and to create pathways for distributing negative pressure between the inner surface of the chamber and the wound. The kit for wound treatment may further comprise at least one tube having a first end connected to the chamber, the at least one tube being in fluid communication with the isolated treatment space so as to enable at least one selected from the group of: applying negative pressure to the isolated treatment space and applying a therapeutic agent to the wound, and a therapeutic agent comprising an antibiotic formulated at a concentration of up to or at least about 1000 x MIC with a gel.

In some embodiments, the therapeutic agent further comprises an analgesic.

In some embodiments, the therapeutic agent formulation includes saline.

In some embodiments, the structures have a height of about 0.2 mm to about 5 mm and are spaced about 0.2 mm to about 10 mm from one another.

In some embodiments, the structures intrude into the isolated treatment space in a direction generally perpendicular to the inner surface of the chamber.

In some embodiments, each embossed structure has a shape selected from the group consisting of a cone, a pyramid, a pentagon, a hexagon, a half sphere, a dome, a rod, an elongated ridge with round sides, and an elongated ridge with square sides.

In some embodiments, the chamber is made of a substantially impermeable material.

In some embodiments, the chamber is made of a substantially transparent material.

In some embodiments, the at least one tube is further configured to remove wound fluid from the treatment space. In some embodiments, the kit further comprises a wound fluid collection device housing an absorbant material fluidly connected to the treatment space via the at least one tube. In some embodiments, the collection device includes at least one of: an inline pump, a check valve, and a safety transducer.

In some embodiments, the kit further comprises a source of negative pressure in communication with the chamber via the at least one tube.

In some embodiments, the kit further comprises instructions for use of the device and the therapeutic agent for wound treatment.

In some embodiments, the gel is a hydrogel.

A wound treatment system may comprise a wound treatment device comprising a chamber that includes an inner surface and a sealing portion that defines an isolated treatment space, a plurality of embossed structures arranged in a pattern on the inner surface of the chamber, the structures configured to directly contact a wound and to create pathways for distributing negative pressure between the inner surface of the chamber and the wound, and at least one tube having a first end connected to the chamber, the at least one tube being in fluid communication with the isolated treatment space so as to enable at least one selected from the group of: applying negative pressure to the isolated treatment space and applying a therapeutic agent to the wound, and a wound fluid collection device housing an absorbant material and connected to the isolated treatment space via the at least one tube.

The collection device may include at least one of an inline pump, a check valve, and a safety transducer.

The wound treatment system may further comprise a source of negative pressure in communication with the chamber via the at least one tube.

A method of treating a wound may comprise applying a wound treatment device to the wound to form an isolated treatment space, and delivering at least one therapeutic agent comprising an antibiotic formulated at a concentration of up to or at least about 1000 x MIC with a gel to the isolated treatment space.

The therapeutic agent may further comprise an analgesic.

The method may further comprise subjecting the wound to negative pressure wound therapy via the device.

The method may further comprise debriding the wound.

The gel may be a hydrogel.

The foregoing and other objects and advantages of the disclosure will appear in the detailed description that follows. In the description, reference is made to the accompanying drawings that illustrate a non-limiting preferred embodiment of the invention.

In the drawings, like reference characters generally refer to the same parts throughout the different views. Also, the drawings are not necessarily to scale, emphasis instead generally being placed upon illustrating the principles of the invention and are not intended as a definition of the limits of the invention. For purposes of clarity, not every component may be labeled in every drawing. In the following description, various embodiments of the present invention are described with reference to the following drawings, in which:
FIG. 1 is a perspective view of a wound chamber treatment device with a tube leading from a chamber to a suction source;
FIG. 2 is a side sectional view of the device in FIG. 1;
FIG. 3 is a sectional view of the device in FIG. 1 with an additional tube leading to a port;
FIG. 4 is a sectional view of the device in FIG. 1 with a branching tube leading to a port;
FIG. 5 is a perspective view of the end of the tube communicating with the interior chamber space;
FIG. 6 is a side sectional view of structures engineered on and into the interior surface of the chamber wall, where the structures are of uniform size and shape, and are spaced uniformly apart;
FIGS. 6a, 6b, and 6c present schematics of patterned engineered structures in accordance with one or more embodiments;
FIG. 7 is a side sectional view of two groups of structures engineered on and into the interior surface of the chamber wall, where one group intrudes into the chamber space, the other group intrudes to a lesser extent, and structures from these groups alternate in a regular pattern;
FIG. 8 is a side sectional view of three groups of structures engineered on and into the interior surface of the chamber wall, where such groups have varying degrees of intrusion into the chamber space and alternate in a regular pattern;
FIG. 9a is an overview of structures engineered on and into the interior surface of the chamber wall, where the structures consist of raised ridges;
FIG. 9b is a side sectional view of the raised ridges of FIG. 9a with rounded edges;
FIG. 9c is a side sectional view of the raised ridges of FIG. 9a with square cross sections;
FIG. 10 is an overview of the raised ridge structures shown in FIG. 9a, with the addition of raised dome structures positioned among the ridges;
FIG. 11 is an overview of raised ridge structures engineered on and into the interior surface of the chamber wall, where two parallel lines of such structures form a channel;
FIG. 12 is an overview of raised dome structures engineered on and into the interior surface of the chamber wall, where two parallel lines of such structures form a channel;
FIG. 13 is a view of a wound chamber, showing a pattern of channels leading to the center of the chamber and then to the tube communicating from the interior of the chamber space;
FIG. 14 is a view of a radiating pattern of channels leading to the communicating tube;
FIG. 15 is a view of a branching pattern of channels leading to the communicating tube;
FIG. 16 is a view of a sub-branching pattern of channels leading to the communicating tube;
FIG. 17 is a side sectional view of a fold in the chamber wall;
FIG. 18a is a side sectional view of a fold in the chamber wall, with structures engineered on and into the inner surface of the fold, which structures maintain continuous open space within the fold;
FIG. 18b is a side sectional view of the fold in the chamber wall of FIG. 17 with structures engineered on the inner surface of the fold;
FIG. 19 is a view of a wound chamber configured as a tube for placement over a limb, and having engineered structures and channels on the interior surface of the chamber wall;
FIG. 20 is a sectional view of the device in FIG. 1 showing a fluid collector placed before the suction source;
FIG. 21 is a sectional view of a suction device in the form of a squeeze bulb of deformable material;
FIG. 22 is a sectional view of a suction device in the form of a flexible chamber containing one or more compression springs;
FIG. 23 is a sectional view of a suction device in the form of a wedge-shaped chamber containing one or more torsional springs;
FIG. 24 is a sectional view of the device in FIG. 23 containing a flat spring;
FIG. 25 is a sectional view of a suction device with a trap and filter incorporated into the exhaust port;
FIG. 26 is a perspective view of a wound chamber treatment device for wounds on the face, head, and neck in accordance with one or more embodiments;
FIG. 27 is a perspective view of another embodiment of a wound chamber treatment device for wounds on the face, head, and neck in accordance with one or more embodiments;
FIG. 28 is a perspective view of an unassembled wound chamber treatment device for wounds on the face, head, and neck in accordance with one or more embodiments;
FIG. 29 is a front view of an assembled wound chamber treatment device for wounds on the face, head, and neck in accordance with one or more embodiments;
FIG. 30 is a perspective view of a wound fluid collection device in accordance with one or more embodiments;
FIG. 31 is a schematic side view of a wound fluid collection device in accordance with one or more embodiments;
FIG. 32 is an exploded schematic side view of a wound fluid collection device in accordance with one or more embodiments;
FIG. 33a is a perspective view of a wound chamber treatment device in accordance with one or more embodiments;
FIG. 33b is a side view of a wound chamber treatment device in accordance with one or more embodiments; and
FIGS. 34a-35d present data discussed in the accompanying Examples.

While the invention is susceptible to various modifications and alternative forms, specific embodiments thereof have been shown by way of example in the drawings and are described below in detail. It should be understood, however, that the description of specific embodiments is not intended to limit the invention to the particular forms disclosed, but on the contrary, the intention is to cover all modifications, equivalents, and alternatives falling within the scope of the invention as defined by the appended claims.

### DETAILED DESCRIPTION

The present disclosure is directed to providing a simple, safe, disposable, and cost-effective device that is easy to install and operate, that allows freedom of motion to the patient, and that overcomes, or at least reduces the effects of, one or more of the problems set forth above. In at least some embodiments, the present disclosure does not require the use of an interface layer such as a porous insert. In some embodiments, a one-piece, two-piece, or multi-piece construction of the device is suitable for patient treatment and eliminates virtually all leaks, therefore preserving and maintaining negative pressure within the wound without the need for constant or frequent regeneration of negative pressure. In addition, the structure of the device is configured to promote wound healing and to create pathways through which negative pressure can be distributed and maintained in the treatment space. The device may contact the wound directly, without the use of an interface layer such as a porous insert. In some embodiments, the device may be used in conjunction with a sustained-release therapeutic agent. The therapeutic agents may be formulated as described further herein, such as with a biomaterial. The biomaterial may be naturally occurring and biocompatible. In some embodiments, the biomaterial may be cross-linked with the therapeutic agent. The biomaterial may be selected based on its properties. In some embodiments, the biomaterial may be, for example, a hydrogel, a hydrocolloid, alginate, or any other gel. The device may be made of a substantially impermeable material such as to promote healing and to facilitate the use of therapeutic agents. A wound exudate collection device may interface with and be used in conjunction with the treatment device. The indications for the present disclosure may be expanded beyond the limitations imposed on current devices. The cost-effectiveness of the present disclosure may lead to the provision of negative pressure wound therapy on a more widespread basis and earlier in the timeline of wound care.

In accordance with various aspects and embodiments, the devices and methods of the present disclosure may be used to treat full and partial thickness burns, traumatic wounds, post surgical wounds, infected wounds, post infection wounds, skin loss due to dermatological conditions, and other conditions that result in skin and deep tissue loss. In some non-limiting embodiments, a patient may have a total body surface area (TBSA) injury of about 5% to about 30% or greater. In some embodiments, the wound may be a bilateral wound. The wounds may be acute or chronic. In at least some embodiments, deep dermis, subcutaneous tissue, muscle, fascia, tendon or bone may be exposed due to the nature of the wound. The devices and methods may be used anywhere on the human body, and may also be suitable for veterinary applications. The devices and methods may, for example, be used for wound preparation to prevent scarring as well as the fixation and protection of skin grafts during wound treatment. In some specific non-limiting embodiments discussed herein, wound treatment may relate to injuries occurring on the head, face, and/or neck. For example, a patient's cheeks, forehead, and/or crown may be treated. The head including facial tissue presents unique challenges which may be addressed in accordance with one or more embodiments.

One aspect of the present disclosure is seen in a wound treatment device including a chamber defining a treatment space around the wound. The flexible adhesive base of the chamber forms a water-tight and gas-tight seal. A tube communicates from the treatment space to a source of suction. In at least some embodiments described herein, the source of suction is capable of generating and/or maintaining sub-atmospheric pressure within an enclosed space. The suction source also serves as a receptacle for materials removed from the chamber, including wound fluid. All components preferably are inexpensive, lightweight, and disposable.

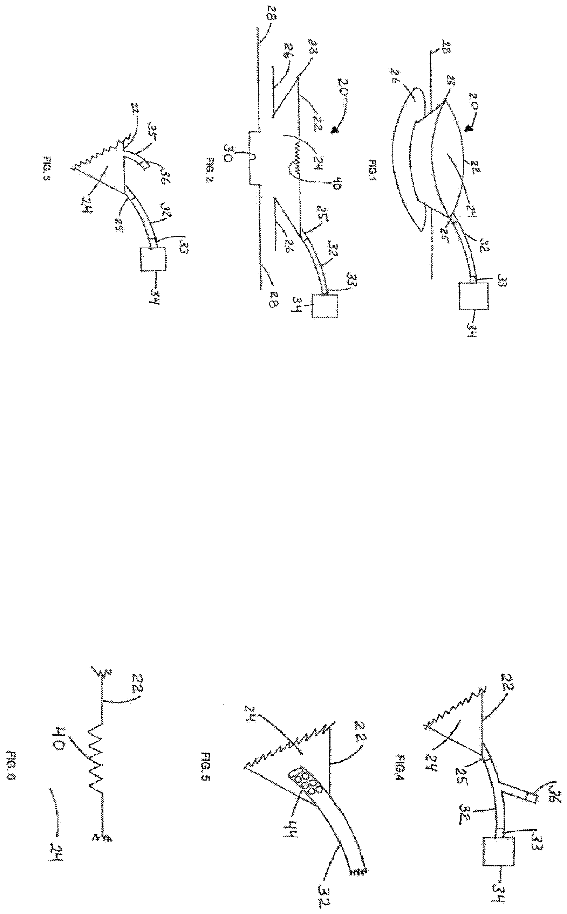

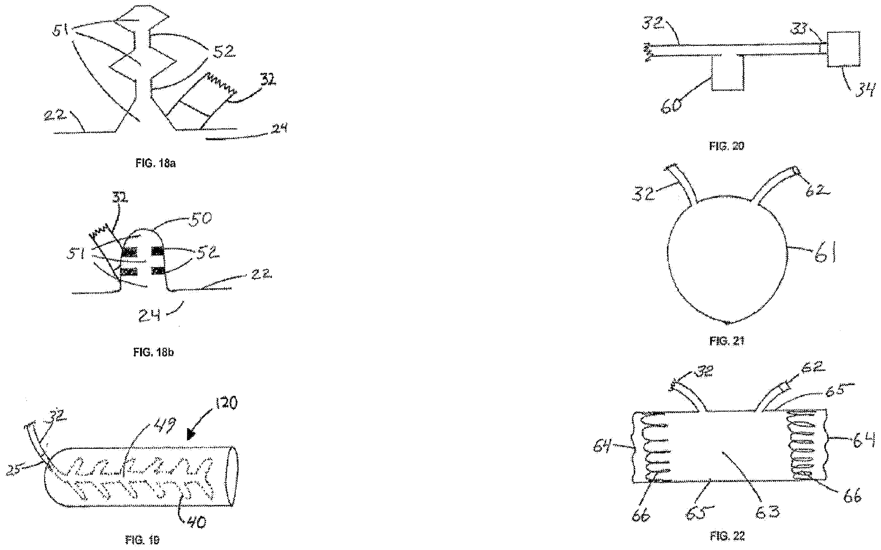

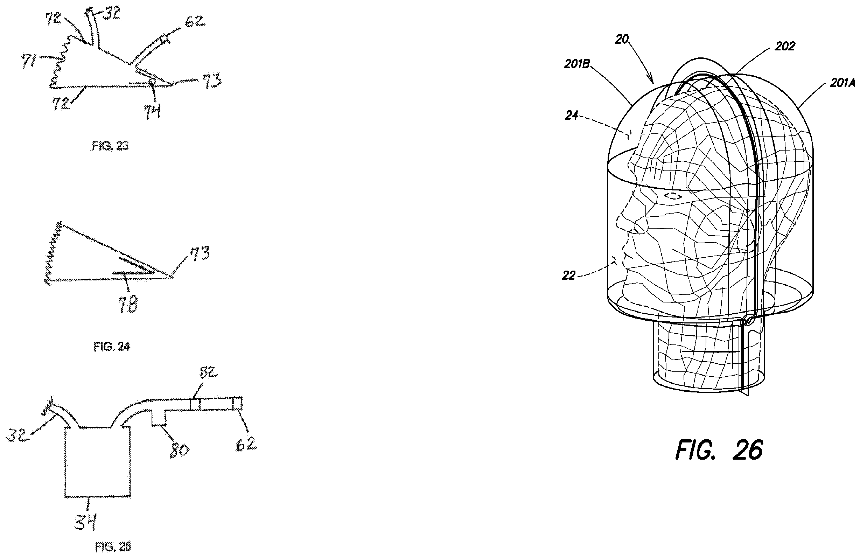

Referring to FIGS. 1 and 2, views of a wound treatment device 20 are provided. The device 20 includes a chamber 22 defining a treatment space 24 and a base 26 that may be sealed to a skin surface 28 of a patient over a wound 30. In the illustrated embodiment, the chamber 22 has a bellows configuration with a fold 23. However, the invention is not so limited, and other configurations of a chamber formed of a flexible, moisture and gas impermeable material may be used. The chamber may also be transparent to allow for visual inspection of the wound during treatment. In accordance with certain embodiments, the material is substantially transparent. In other embodiments, the material may be substantially opaque. The use of an impermeable material is particularly advantageous for introducing therapeutic agents to the wound. Additionally, the impermeable nature of the chamber material prevents water loss from the wound, which facilitates improved healing. Materials from which the device 20 may be made will be discussed in further detail below. The device 20 can be designed for use with any wound on any body part, including both human and veterinary applications. Various geometries such as circular, square, rectangular, tubular, pouch, envelope or other shapes may be implemented based on the intended application. In accordance with some embodiments, chamber 22 defining treatment space 24 may be configured for a specific body part. For example, a chamber in the form of a tube or sleeve for placement over a limb is shown in FIG. 19, whereas a chamber in the form of a hood for placement over a head is shown in FIG. 26 as described further below.

In accordance with one or more embodiments, the geometry of the wound treatment device chamber may generally involve a degree of convexity or concavity in order to enable it to be pulled down, for example, into contact with a deep or full thickness wound, such as upon application of negative pressure wound therapy. In some non-limiting embodiments, the material used to form the chamber and/or its shape may impart this design in terms of conforming to anatomical and/or wound contours. In some embodiments, the device may be a concave structure that is generally hallowed inward towards a wound. A major redundancy (concavity with respect to the device) of the construction is highly desirable in certain cases such as deep wounds. In a deep wound, the underside of a generally concave device may be pulled into every deep part of the wound in order to provide negative pressure wound therapy and micromechanical forces to the entire deep wound surface. An additional reason for redundancy in the construction of the device is to provide folds that extend into the deep part of the wound, thus creating channels for air and fluid flow.

Still referring to FIGS. 1 and 2, a dermal or cutaneous adhesive material may be provided on a bottom surface of the base 26 for providing a fluid-tight seal with sufficient adhesive strength to prevent inadvertent removal of the chamber 22 or breach of the fluid-tight seal during normal patient movement. Numerous adhesive materials sufficient for these purposes are known to those of ordinary skill in the art.

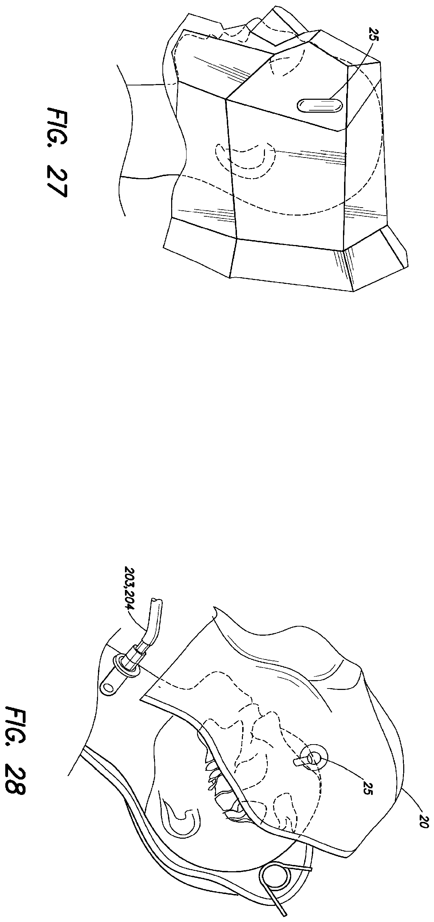

In accordance with some embodiments, device 20 can be specifically designed for treating wounds occurring on the head, face, and/or neck. The device may cover only the head, or both the head and neck. Referring again to FIG. 26, a chamber may be formed that fits over the head and attaches at the base of the neck for example at, or proximate, the collar bone. In some embodiments, the chamber may be defined by a single piece of material, or a two or more piece design may be implemented. The device may be oversized to fit any size head or may be customized. Oversized devices may lead to the presence of wrinkles in use which may be advantageous for negative pressure distribution. In accordance with other embodiments and referring to FIG. 26, the device may be comprised of two pieces 201A and 201B that may adhesively join to form a seam 202, for example, around the midline of the head and across the ears. In some embodiments, the pieces may be joined by other methods, and may for example, by a zipper type or ziploc seal. Still, in accordance with other embodiments and as shown in FIG. 27, the device may be made from a plurality of pieces that contour the face.

The head device may or may not have openings for the nose and mouth. If the device is constructed to cover the nose and mouth, the nose and mouth may be surgically sealed for treatment. For example, soft conforming obturators, or alternatively, sutures may be used. Referring to FIG. 28, a patient being treated with a device 20 covering the nose and mouth may breathe via an installed tracheostomy tube 203 and be fed via an installed gastrostomy tube 204. These tubes may be fitted outside of the device, for example, at the neck. Contact lenses (not shown), such as oversized lenses, may be used to protect the eyeballs during treatment.

A tube 32 is attached to the chamber 22 preferably at a location spaced above the base 26 and communicates with the treatment space 24. The tube 32 is constructed to maintain its shape without collapsing and to permit the passage of wound fluids and wound debris. The tube 32 may be permanently fixed to the chamber 22, or a fitting, such as a tubular port 25 may be provided to allow the attachment and removal of the tube 32 or any other device that can deliver material or therapeutic agents to, or remove material from, the treatment space 24. The tube 32 may terminate at a wall of the chamber 22, or it may extend through the wall a distance and terminate within the treatment space 24, where it may communicate with such space, with channels formed on the inner surface of the chamber wall, or with folds formed in the chamber wall. The tube 32 is sealed to the chamber 22 in such a manner as to prevent the escape of liquid or gas from the treatment space 24 to the outside environment. A distal end of the tube 32 terminates at a device that generates sub-atmospheric pressure, such as suction device 34. The suction device 34 may be a pump, although other types of devices may be used as discussed below. A fitting 33 may be provided to permit the detachment and reattachment of a suction device 34 to the tube 32.

Referring to FIG. 28, when device 20 is used to treat face, head, and/or neck wounds, the device 20 may comprise a plurality of tubular ports 25 for communication with one or more tubes 32. The ports 25 may be strategically placed based on the geometry of the chamber. At least one tubular port 25 may be located at the top of the device and at least one tubular port 25 may be located at the bottom of the device. In accordance with some embodiments, tubular ports 25 may be positioned on the side of the patient's face, at the temples proximate the ears. In some embodiments, there are at least two tubular ports present.

Turning to FIG. 3, a sectional view of the device 20 is provided, showing a second tube 35 attached to the chamber 22 and communicating with the treatment space 24, with channels, or with folds. A distal end of the tube 35 terminates in a portal 36. The disclosure is not limited to any number of communicating tubes, and multiple tubes and portals may be provided for accessing the treatment space 24. FIG. 4 shows the device in FIG. 1 with a branch of the tube 32 that leads to a portal 36. The portal 36 may be used for the delivery of therapeutic agents-such as antimicrobials, antibiotics, antifungals, and analgesics-prior to, during, or after the delivery of negative pressure. As such, the portal 36 may be a lure configured for attaching to a container or a syringe. Alternatively, therapeutic agents may be delivered through the same tube 32 that communicates with the suction device 34.

In accordance with some embodiments, the device may allow for the delivery of a therapeutic agent directly to the wound. As such, the therapeutic agents can be delivered in concentrations significantly higher than could otherwise be administered intravenously or orally. For example, antibiotics can be applied directly to the wound at concentrations in a range from about the conventional oral concentration to up to about 1000 times the conventional oral concentration (i.e., 1000 x MIC), or even higher. If ingested or administered directly to the bloodstream, these concentrations would be toxic to the body. Topical application facilitates the use of significantly higher concentrations that facilitate healing. Combinations of therapeutic agents, such as analgesics, antibiotics, and chemical or enzymatic debriding agents may also be used, and may advantageously reduce or eliminate the need for surgical debridement of the wound. The therapeutic agents may be delivered in concentrations of 1 times MIC to at least 1,000 times MIC. In some embodiments, concentrations of up to 5,000 times MIC during shorter periods of treatment may be used. In large wounds, for example, a limiting factor may be systemic toxicity from absorption from the wound. Because of the combination of half-life absorption and surface area, the total amount of pharmacologic agent should generally be limited to 5 times a standard total IV dose in a 24 hour period. In some non-limiting embodiments, concentrations of 1,000 to 5000 X MIC may be safely and effectively administered. In other non-limiting embodiments, 15 to 500 to 1000 X MIC concentrations may be safely and effectively administered and may find particular utility, for example, in various military applications. Concentration, volume, absorption rate, and surface area may all be considerations and defined variables in terms of targeted and accurate delivery of various therapeutic agents in accordance with various embodiments. In various embodiments, a wound treatment device as described herein may be used to form a reservoir of a formulated therapeutic agent to promote availability for healing. Use of the devices in combination with formulated therapeutic agents described herein may demonstrate a synergistic effect in terms of efficiency in wound healing.

In accordance with one or more embodiments, one or more therapeutic agents may be formulated and delivered for improved efficacy and/or usability in connection with a wound therapy device. Analgesics and/or antibiotics and/or antifungals and/or debriding chemicals and/or anti-inflammatory agents and/or scar-reducing agents and/or chemotherapeutic agents may be delivered to a wound site, whether small molecules or macromolecules. In some embodiments, therapeutic agents may be formulated for sustained-release to enable prolonged care. Maintenance of desired placement of therapeutic agents relative to a wound may also be promoted by their formulation as described herein. High-dose topical treatment of wounds, not possible systemically, may be enabled via delivery of active treatment agents in liquid or gel form to the device. In some embodiments, an antibiotic may be gentamicin, vancomycin, clindamycin, minocycline, or tetracycline. In some embodiments, an anti-fungal may be diflucan or amphotericin. In some embodiments, an analgesic may be lidocaine. In some embodiments, an anti-inflammatory agent may be a steroid or a non-steroidal anti-inflammatory, such as indomethacin or aspirin. In some embodiments, a scar-reducing agent may be cortisone. In some embodiments, a chemotherapeutic agent may be 5FU. One or more of these and other various therapeutic agents may be implemented alone or in combination.

In some embodiments, one or more therapeutic agents or combination thereof may be formulated with a gel for delivery to a wound site. As discussed, herein, a hydrogel, hydrocolloid, alginate, or any other gel may be used. The formulation can be formed and then delivered to the wound treatment chamber. Alternatively, a gel may be positioned within the wound treatment chamber prior to the delivery of one or more therapeutic agents. Sustained-release delivery of one or more therapeutic agents from a reservoir of a gel may be provided. A layer of gel, such as a layer which is 2 to 10 mm thick, may be applied. In one embodiment, a 3 mm thick layer of gel may be used, and the total amount of gel may be 100ml (100cc). When using, for instance, Vancomicin or Gentamicin, the MIC is typically around 2 micrograms per ml or cc for common bacteria. A total of 200 mg of each of these antibiotics may be required in 100 ml of gel. For purposes of example only, an upper arm which has a surface area of approximately 2,000 square centimeters would use 60 cc's gel based on a 3mm thick layer.

A hydrogel is typically a three-dimensionally cross-linked network composed of hydrophilic polymers with high water content. A hydrogel, gel, hydrocolloid, alginate, methyl cellulose, gelatin or any other gel may impart sustained-release characteristics to the therapeutic agent and may also promote the staying in place of the therapeutic agent relative to the wound being treated. For example, the gel may allow the therapeutic agent to be released for at least 24 hours, for at least 48 hours, for at least 72 hours, or for at least 96 hours. The half-life of active antibiotics may be approximately 24 hours, 48 hours, 96 hours, or longer in connection with embodiments involving sustained release formulations of a gel and/or antibiotic and/or analgesic. The drug release kinetics may be controlled by diffusion of the drug through the network. The polymer concentration and molecular weight, and type and extent of chemical and physical crosslinking allow for manipulation of the physical properties of the gel, such as viscoelasticity and drug release kinetics. Alginate, for example, may be used in various formulations of therapeutic agent(s) to achieve an acceptable viscosity range. Relatively high viscosity may be a desirable property to facilitate treatment, as well as relatively low freezing point.

The gel may be selected for at least one of its properties. For example, the gel may be selected for its pre-gel rheological properties, mechanical stability post-gelation, or control over the release of the incorporated therapeutic agent. Prior to gelation, the gel precursor may have sufficiently low viscosity to allow it to flow into molds and conform to the skin-facing side of the device. Subsequent to gelation, the gel must possess appropriate stiffness to maintain its form and not allow leakage from the device if it is ruptured, and must possess sufficient toughness to prevent its fracture with handling of the device.

According to Table 1, gel-therapeutic agent preparations comprising an antibiotic and a 0.5% (agarose) hydrogel may be stable for up to one week. Drugs dissolve passively into the gel and stay stable over time. In some embodiments, the gel-therapeutic agent preparation may further comprise saline. The gel may be biocompatible, meaning that it is structurally similar to the extracellular matrix in tissues. The gel may be flowable, allowing either for, for example, casting into a mold or injection via needle through a port. The loading of the hydrogen-therapeutic agent preparation in the device may allow consistent and uniform contact with the skin even with patient movement, maintaining continuous delivery of the therapeutic agent to the underlying tissue. The gel-therapeutic agent preparation may prevent loss of the therapeutic agent in the event of a minor leak in the adhesive of the device.

**Table 1**

| | | | |
|---|---|---|---|
| | | | |

| **Antibiotic** | **Original concentration (µg/ml)** | **Drug activity (µg/ml)** | |
|---|---|---|---|
| | | **3 days** | **7 days** |
| Gentamicin | 2.5 | 2.41 | 2.04 |
| Minocycline | 9.0 | 8.86 | 8.53 |

In some specific non-limiting embodiments, the gel may comprise a naturally derived polysaccharide. In some embodiments, the gel may comprise alginate, agarose, hydrocolloid, or cellulose or a combination thereof. In at least some embodiments, the gel may be a hydrogel.

In at least some embodiments, both an antibiotic and analgesic to reduce or eradicate infection and to ease pain may be implemented.

In some embodiments, the gel may be lyophilized prior to or subsequent to loading in the device to minimize weight, and facilitate storage, stability, and transport.

In accordance with one or more embodiments, the engineered skin-facing surface of the device chamber (embossed pattern as described further below) may promote even distribution of a gel formulated therapeutic agents. Devices as described herein may also facilitate slow or sustained release of therapeutic agent.

In accordance with one or more embodiments, wound treatment devices may provide a sterile enclosure that can be applied immediately after injury to provide a protective dressing and a tool for precise topical delivery of therapeutic agents. The device can also serve as a wound incubator for strategic tissue regeneration in a controlled environment. The device is therefore capable of addressing problems of wound depth progression, infection and sepsis from first treatment throughout the treatment process in both military and civilian populations. Prolonged field care and/or evacuations can beneficially be safely and comfortably endured until an advanced medical facility is reached. Rapid decontamination, reduced pain, reduced inflammation, reduced tissue loss, less scarring, and improved quality of healing may all be promoted.

In some embodiments, the device may be applied to a fresh wound within 24 hours of injury. In accordance with one or more embodiments, the device may remain in place for up to about four to seven days or longer before being replaced or removed entirely. Wound fluid may be removed and therapeutic agent a gel may be added to the device via a port. A transparent device may allow for wound evaluation. A sleeve may be placed over the device for additional protection. The device may be suitable for single-use sterile packaging for disposable one-time use. The gel may be rehydrated after storage.

In accordance with one or more embodiments, combination and/or serial treatment regimens may be practiced. The devices may be applied after hemostasis. A wound may be debrided for a period of time, and then subjected to treatment with the devices and formulated therapeutic agents described herein. In some non-limiting embodiments, the disclosed therapeutic agents may be left in place on the wound for up to a week or more prior to further evaluating the wound to discern further treatment steps. Beneficially, the wound may be left in the sterile environment of the disclosed devices for extended periods of time to facilitate healing. Furthermore, the formulated therapeutic agents also promote relatively uninterrupted periods of wound therapy. In some embodiments, periods of negative pressure wound therapy may be applied to the wound via the disclosed devices as described herein. Such therapy may be continuous or periodic, and may be performed simultaneously, prior to, or subsequent to application of the formulated therapeutic agents. In at least some embodiments, negative pressure therapy may be used sequentially with drug delivery. In certain embodiments, negative pressure therapy is not administered in parallel with drug delivery as may be an approach distinct from, for example, conventional wound irrigation techniques.

Turning now to FIG. 5, the end of the tube 32 extending into the chamber space 24 is shown with multiple holes 44. The purpose of the holes 44 is to ensure that gases, liquids, wound fluid, debris, and other materials can flow and move out of the chamber space 24 into the tube 32 without impediment.

The wound may advantageously be monitored through the substantially transparent chamber material. The negative pressure device 20 may also be equipped with sensors to monitor certain parameters within the chamber space 24. For example, oxygen, carbon dioxide, pH, temperature, and other parameters may be measured and monitored.

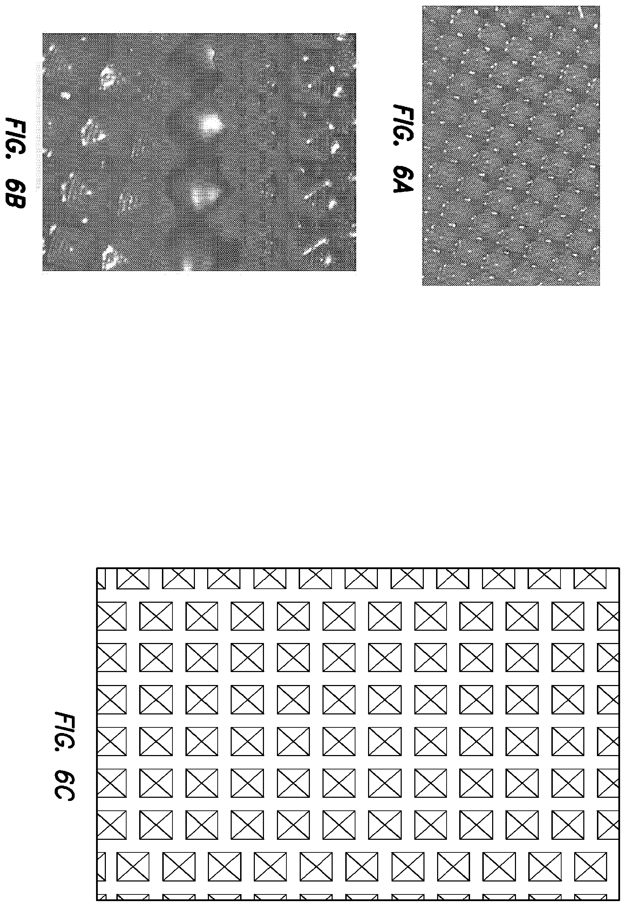

Referring to FIG. 6, the interior surfaces of the chamber wall may be configured with structures 40 that are engineered on the surfaces. FIGS. 6a-6c present schematics of different patterned engineered structures in accordance with one or more non-limiting embodiments. The portions of the interior surfaces with engineered structures 40 may be varied from that shown in the figures, and preferably a high percentage of the interior surfaces include engineered structures 40. The structures preferably cover at least 50% of the interior surfaces, and more preferably at least about 95% of the interior surfaces. These structures are raised when viewed from within the chamber space 24, and they intrude into such space in directions generally perpendicular to the interior surfaces of the chamber space 24. These structures can be any shape, including without limitation a cone, a pyramid, a pentagon, a hexagon, a half sphere, a dome, a rod, an elongated ridge with rounded sides, or an elongated ridge with square sides. The structures can be provided as identical shapes, or in any combination of shapes. The structures can be provided with identical sizes, or in any combination of different sizes. The structures can be provided in a regular or irregular pattern on the surface. The distance of intrusion into the chamber treatment space 24 from the chamber wall by such structures (height of such structures) is preferably between 0.01 mm and 20 mm, preferably between 1 mm and 1 cm, and most preferably about 2 mm. The spacing between such structures is preferably between 0.01 mm and 5 cm, and the spacing for example, is most preferably about 2 mm apart. In some embodiments, about 2 mm high structures are arranged about 2 mm apart. When larger structures are used, the structures may be spaced further apart and when smaller structures are used, the structures may be spaced closer together. For example, a configuration of pyramids of 0.2 mm in height may be spaced about 0.2 mm apart, whereas a configuration of larger pyramids of about 5 mm high may be spaced 10 mm apart.

The engineered structures 40 interface with the wound surface during use of the device 20. The engineered structures may directly contact the wound surface. One purpose of these structures is to ensure that negative pressure established within the chamber space 24 is evenly distributed and maintained throughout such space. As negative pressure is established within the tube that leads to the source of suction or sub-atmospheric pressure, the chamber will lie tighter against the wound tissue. The device 20 includes the engineered structures 40 in order to define pathways to establish, distribute, and maintain negative pressure across the wound surface and prevent complete contact between the inner surfaces of the chamber and the wound tissue. Without such structures, the chamber wall would make complete contact with the wound surface. As a result, there would be no space within which negative pressure could be established, distributed, and maintained. Therefore, the engineered structures are preferably semi-rigid. The term "semi-rigid" should be understood as meaning that deformation only occurs at a microscopic level under operating negative pressures in the range of 0.5-2 psi. Alternatively, the engineered structures may be somewhat flexible depending on the spacing between the structures. In addition, the structures are engineered to reduce the extent to which wound tissue can enter the space between the structures, so that a sufficient amount of open space is maintained. The engineered structures may be strategically patterned on the surface to produce desired negative pressure pathways within the chamber.

An additional purpose of these structures is to serve as a form of stimulation to the wound to produce beneficial results, including without limitation the formation of granulation tissue and an increase of micromechanical forces. Such mechanical forces provide stimulation to a portion of the wound tissue, which has been suggested as a contributing factor to the effectiveness of negative pressure wound therapy. From the above discussion and the figures, it should be understood that the flexible chamber is movable over a range of positions. The range of positions includes a first position, such as the position shown in FIGS. 1 and 2, in which the engineered structures 40 are spaced apart from the opening of the chamber defined by the base 26. The range of positions also includes a second position in which at least some of the engineered structures 40 are positioned in the opening of the chamber. The second position is preferably a position in which the engineered structures 40 engage the wound.

The chamber wall can be formed of any appropriate medical grade material that has the following characteristics: flexibility, conformability, gas impermeability, liquid impermeability, the ability to be formed, tooled, and engineered, and the ability to retain the shape, function, and effectiveness of raised structures under desired ranges of negative pressure. The material should generally deter adhesion and in-growth. The material is preferably transparent to allow visual inspection of the wound during treatment. In addition, the material is preferably hypo-allergenic and provided to a medical facility in a sterile condition. For example, the chamber device may be made of a flexible, conformable material such as polyurethane, polyethylene, or silicone, although other similar materials may also be used. The material may have a thickness in the range of about 5 mm to about 100 mm. In some embodiments, the material may have a thickness of from about 1 mil up to about 100 mil. In some specific embodiments, a 5 mil polyurethane membrane may be used to form the treatment chamber.

The chamber is preferably designed to provide sufficient material to lie against the surface of the wound tissue without special sizing, trimming, or other customizing operations. The chamber may be made from a single ply of material, or may be constructed of multiple layers of material in and on which the structures are engineered. It should be understood that a single ply chamber may be made of multiple sheets of material during manufacturing, but is provided to a medical facility in a state in which the multiple sheets are bonded or otherwise connected to one another. For example, individual three dimensional shapes may be adhered or bonded to the inner surface of the chamber wall during manufacturing to provide the engineered structures. A single ply chamber could also be formed from a single sheet of material that defines both the chamber walls and the engineered structures. For example, the engineered structures may be embossed. Alternatively, a multiple layer chamber is provided to a medical facility in a state in which layers of material are stacked to form the chamber. For example, the layer facing the interior treatment space of the chamber could be a layer containing engineered structures that is bonded onto a generally flat layer of material (or multiple sheets of generally flat layers) by a medical practitioner.

The engineered structures can be made by techniques familiar to those in the art, such as embossing, stamping, molding, forming, or bonding. If the structures are created by embossing their shape into the material, the embossed structures may be left in a concave state relative to the outside of the chamber as shown in FIG. 6. Embossed structures may also be formed on a single ply of material that also forms the walls of the chamber and the base. This may provide a chamber that is relatively flexible with semi-rigid structures on a single ply of material. Alternatively, the cavities may be filled with a suitable material to render the structures solid. As another alternative, solid structures can be affixed to the inner surfaces of the chamber.

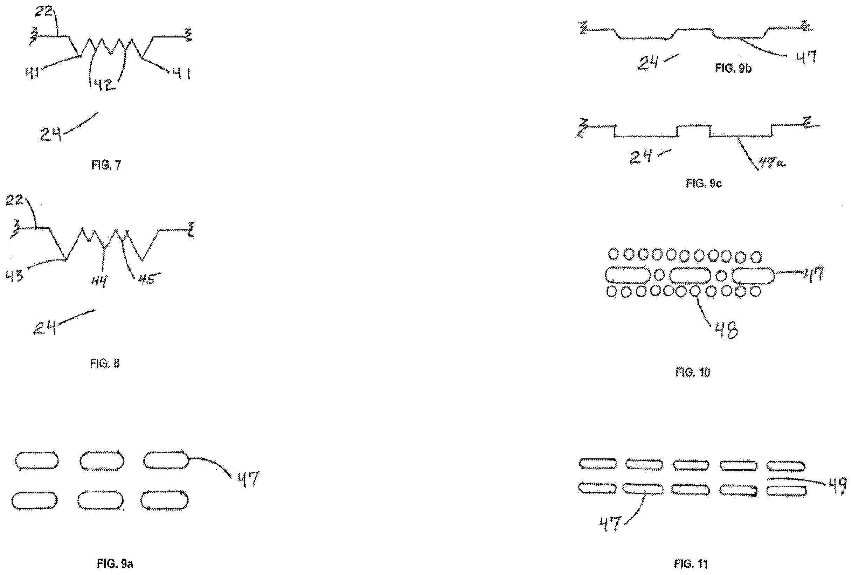

The raised structures on the inner surfaces of the chamber wall can be configured and distributed in a number of patterns. For example, FIG. 6 is a side sectional view of a portion of a chamber wall, showing engineered structures 40 on the interior surface of the material that faces treatment space 24. Structures 40 are identical in shape and size, and are positioned uniformly apart from one another. As another example, FIG. 7 is a side sectional view showing engineered structures 41 and 42 intruding into the chamber space, where structures 41 intrude farther than structures 42, and the structures are configured in a regular alternating pattern of 41-42-41-42 and so forth. As yet another example, FIG. 8 is a side sectional view showing engineered structures 43, 44, and 45 intruding into the chamber space, where structures 43 intrude farther than structures 44 and 45, structures 44 intrude less than structures 43 but farther than structures 45, and structures 45 intrude less than structures 43 and 44. These structures are configured in a regular alternating pattern of 43-45-44-45-43-45-44-45-43 and so forth. The embodiment shown in FIG. 8 makes it difficult for soft wound tissue to penetrate all of the spaces among the raised structures. A sufficient amount of continuous space is established to make possible the distribution of negative pressure, as well as the addition of fluids and therapies and the removal of fluids and materials from the wound. As yet another example, FIG. 9a is an overview of a portion of the chamber wall, showing engineered structures 47 in the form of raised ridges. The engineered structures 47 may be rounded (FIG. 9b), square (FIG. 9c), or a combination thereof when viewed from the side. As yet another example, FIG. 10 is an overview showing engineered dome structures 48 interspersed with ridge structures 47. The engineered dome structures 48 are preferably semi-spherical when viewed from the side, although other shapes are contemplated.

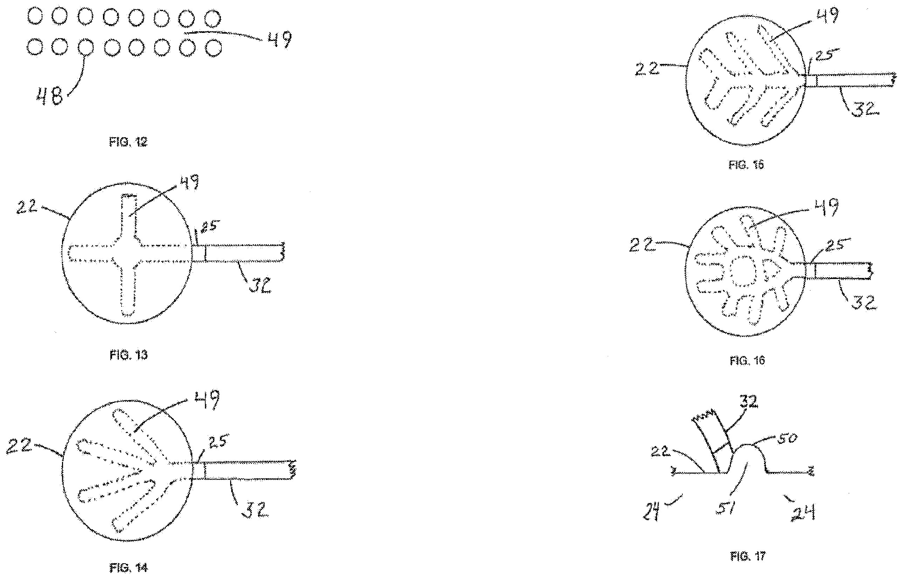

The distribution and maintenance of negative pressure within the chamber device and at all points on the wound may be enhanced by providing defined channel spaces as pathways among the raised engineered structures for the distribution of negative pressure. However, defined channel spaces are not required for providing fluid pathways within the treatment space. FIG. 11 is an overview of a portion of the chamber wall, showing structures 47 arranged in two parallel lines to form channel 49. FIG. 12 shows a channel 49 formed by two parallel lines of raised domed structures 48. Such channels can be configured in various patterns, such as radial, circular, concentric, or branching. FIGS. 13-16 show overviews of patterns of channels 49 leading from tube 32 along the interior surface of chamber 22 facing treatment space 24. For each pattern, the channel 49 defines a space that opens directly to the treatment space 24. The space preferably opens to the treatment space 24 over the entire length of the channel 49.

The distribution and maintenance of negative pressure within the chamber device and at all points on the wound can also be enhanced by the use of folds in the chamber wall to create additional channel space for the distribution of negative pressure. When negative pressure is established within the chamber, the material will tend to fold along the pre-formed location. FIG. 17 shows a channel 50 formed in a fold of the chamber wall. The channel 50 defines a space that opens directly to the treatment space 24. The space preferably opens to the treatment space 24 over the entire length of the channel 50. In order to increase the amount of channel space within such fold, the walls of the fold can be configured with structures that prevent the collapse of such space, and ensure continuous open space for the distribution and maintenance of negative pressure, and the passage of liquid, gas, and other material. As an alternative, FIG. 18a shows engineered structures 52 that prevent the total collapse of the fold, and ensure continuous channel space 51. All channel spaces created on the interior surface of the chamber wall or by means of folds function as means to increase the effectiveness of distributing and maintaining negative pressure within the chamber, and also as means to enhance the effectiveness of removing gas, liquid, wound fluid, debris, and other materials from the chamber treatment space. As another alternative, FIG. 18b shows an embodiment similar to the embodiment shown in FIG. 17 with the addition of engineered raised structures 52 on opposite sides of the fold. The engineered structures 52 are provided so that the fold will not collapse to the point where all of its interior surfaces form a tight seal against the movement of negative pressure. However, some of the interior surfaces, such as those adjacent to the fold, preferably contact the wound to provide stimulation as discussed above. The folds described in the previous embodiments are preferably formed at certain defined areas by molding or embossing the surfaces of the chamber 22.

FIG. 19 shows a wound chamber device 120 for delivering negative pressure and therapeutic substances in the form of a tube that can be placed over a limb. The wound chamber device 120 is generally cylindrical and includes an open end and a closed end, though the chamber may have other shapes to accommodate other body parts, and may for example, be suitable for fitting over the head. The open end is preferably sealed with a cuff or collar (not shown), and the open end may include adhesive on the interior surface. The wound chamber device 120 includes engineered structures 40 and channels 49 on the interior surface of the chamber wall.

As shown in FIG. 20, a fluid collector 60 may be positioned on the tube 32 between the chamber 22 and the suction device 34. The collector 60 is intended to receive fluid extracted from the chamber space 24 and debris or material from the wound and store such materials for eventual disposal. The collector 60 may be detachable from the tube 32, in order to replace a full collector with an empty collector.

Suction for the wound treatment device is provided by a suction device 34, which may be a pump that is connected and disconnected to the chamber device by appropriate connectors to provide sub-atmospheric pressure. Although the wound chamber can be used with a motor driven pump, it is also effective with a hand-powered device actuated by the caregiver or patient. The hand-powered device may be a squeeze bulb that provides suction by means of the energy stored in the material of its construction. Alternatively, the suction device may be powered by springs that are compressed by the user. The springs can be selected to produce the clinically desired level of negative pressure. The amount of suction provided by these suction devices is therefore dependent on the level of force generated by squeezed material or the springs. Unlike a motor driven suction pump, the hand powered device preferably cannot produce a high level of suction that may cause an adverse effect to wound healing.

Referring to FIG. 21, a suction device 61 in the form of a bulb constructed of a deformable material that stores the energy of deformation may be used. The tube 32 communicates with the interior of the suction device 61. A one-way exhaust valve 62 also communicates with the interior of the suction device 61. When the user squeezes the suction device 61, air within the device is expelled through the exhaust valve 62. A portion of the energy used to deform the suction device 61 is stored in the material of which it is constructed, thus maintaining suction within the device, as well as within the tube 32 and the chamber space 24. The bulb is selected and engineered to maintain a constant force and to maintain the clinically desired level of negative pressure within chamber space 24. Fluid from the wound 30 can flow through the tube 32 into the suction device 61 where it can be stored prior to disposal. Once the suction device is full of fluid, the production of negative pressure ceases. The fluid capacity of the suction device thus operates as a safety shut-off mechanism without the need for electronic sensors and controls.

FIG. 22 shows an alternative suction device 63, consisting of flexible sides 64 and rigid sides 65. Compression springs 66 are located within suction device 63. The tube 32 and the exhaust valve 62 both communicate with the interior of the suction device 63. When the user squeezes the rigid sides 65 towards one another, the springs 66 are compressed and air within the device is expelled through a one-way exhaust valve 62 thus maintaining suction within the device, as well as within the tube 32 and the chamber space 24. The springs 66 are selected and engineered to maintain a constant force against rigid sides 65, and to maintain the clinically desired level of negative pressure within chamber space 24. Fluid from the wound 30 can flow through the tube 32 into the suction device 63 where it can be stored prior to disposal of the entire device 63. This suction device will also cease operating when it is filled with fluid.

FIG. 23 shows an alternative suction device 70, consisting of rigid sides 72, joined by hinge 73, and flexible side 71. A torsional spring 74 is attached to either the interior or the exterior of rigid sides 72. The tube 32 and the exhaust valve 62 both communicate with the interior of the suction device 70. When the user squeezes the rigid sides 72 towards one another, the spring 74 is compressed and air within the device is expelled through a one-way exhaust valve 62, thus maintaining negative pressure within the device, as well as within the tube 32 and the chamber space 24. The spring 74 is selected and made to maintain a force against rigid sides 72 to maintain the clinically desired level of negative pressure within chamber space 24. Fluid from the wound 30 can flow through the tube 32 into the suction device 70 where it can be stored prior to disposal of the entire device. FIG. 24 shows the device of FIG. 27 where the torsional spring 74 has been replaced by a flat spring 78.

For the previous suction devices, once suction has been established, fluid may flow from the wound to the suction device, where it may be collected and stored for eventual disposal. Alternatively, a separate fluid collector, such as the fluid collector 60 in FIG. 20, can be positioned between the chamber and the suction device. Once the suction device has expanded to its original shape, suction ceases. The suction device will not continue to operate, and can be disconnected and disposed of. If treatment is to be continued, a new suction device can be connected and activated.

FIG. 25 is a sectional view of a trap 80 and a filter 82 interposed between the suction device 34 and the exhaust valve 62 for the purpose of preventing the expulsion of liquids or aerosols from the suction device.

The present disclosure can be engineered to operate at various levels of negative pressure, in accordance with clinical procedures. Traditional negative pressure wound healing devices can apply a negative pressure of between -0.5 and -2 psi, or about -750mm Hg to about -125 mm Hg. The device of the present disclosure operates efficiently in this range. The chamber material conforms to the shape of the wound, and the embossed projections maintain their shape and functionality. However, the chamber can be engineered to operate at higher levels of negative pressure. The device of the present disclosure may also work efficiently at lesser negative pressures of for example from about -125 mm Hg to about -10 mm Hg. The application of less negative pressure may reduce pain and other complications. In addition, if a hand-powered suction device is used, the operating pressure of the device may be higher than the commonly accepted range; that is, the device may operate at a pressure close to 0 psi before suction ceases.

In accordance with one or more embodiments, a negative pressure wound therapy device may be vacuum-assisted to stimulate blood flow and new blood vessel growth, biomechanically stimulate cells to encourage division and proliferation, and to remove factors that might inhibit healing such as bacteria. Depending on the stage of treatment, connecting tubes can plug into different devices to apply negative pressure, drain a wound, and deliver therapeutic agents.

The present disclosure eliminates many of the drawbacks to existing negative pressure wound therapy systems. For example, the device of the present disclosure is preferably simplified and lightweight, and allows visual inspection of the wound. In some embodiments of the disclosure, the patient is not restricted to a source of electricity or a battery pack. The system can be worn with ease, so that the patient's mobility is not otherwise compromised. In addition, the wound interface appliance can be applied quickly without the need for custom fitting and construction. The device preferably does not leak due to the smooth adhesive base, eliminating the need for constant suction from an electric pump with sophisticated controls and safety measure. There is no interface material such as a porous wound insert that can potentially cause tissue in-growth and harbor infectious material. Instead, the inner surfaces of the chamber are generally non-porous and non-adherent to prevent any interaction with the wound tissue. Further still, the suction pump preferably has built-in safety limitations on force of suction, duration of operation, and overfilling of the collector for wound fluid. The engineered surface may stimulate the wound and create an efficient pathway for the distribution of negative pressure. The wound treatment chamber may be customized for use on any body part including limbs, as well as the head, face and/or neck. The devices and methods disclosed herein may be used in conjunction with conventional wound debridement and grafting techniques without any contraindications. The devices and methods may facilitate the fixation and protection of skin grafts and micrografts. The devices and methods may be superior to conventional approaches to administering negative pressure in terms of at least granulation tissue formation.

In accordance with one or more embodiments, a wound treatment device may be used in conjunction with a wound fluid collection device. A wound treatment system may therefore include a wound treatment device and a wound fluid collection device. The collection device may include an absorbable material, such as an absorbable insert. Any absorbable material, preferably biocompatible in terms of disposability, readily known to those of ordinary skill in the art may be implemented. The absorbable material may at least partially fill a compartment defined by the wound fluid collection device. The collection device insert may absorb wound fluid and package it for disposal during operation. All components preferably are inexpensive, lightweight, and disposable. In at least some embodiments, the wound fluid collection device may operate substantially as a filter or water trap.

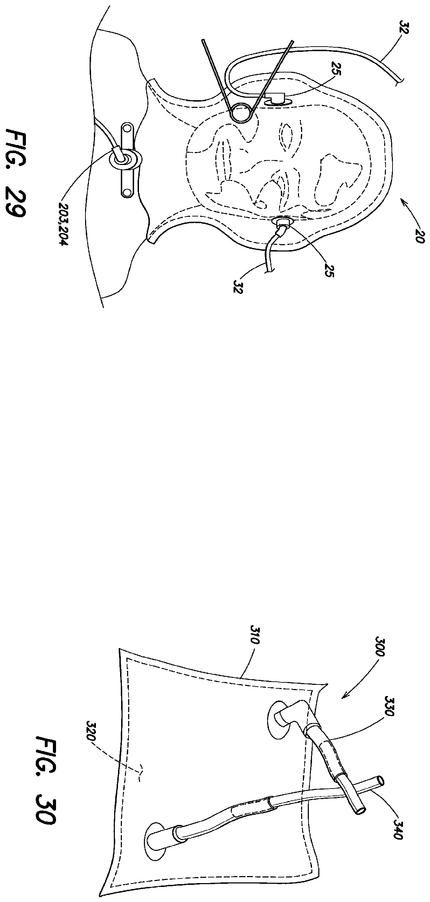

Referring to FIG. 30, a view of a wound fluid collection device 300 is provided. The device is intended to receive fluid, debris, exudate, or other materials removed from a wound during treatment or therapy and store such materials for eventual disposal. The device includes a compartment 310 defining a collection space. In the illustrated embodiment, the compartment has a pillow-shaped configuration. However, the invention is not so limited, and other configurations of a compartment formed of a flexible, moisture and gas impermeable material may be used. The use of an impermeable material is particularly advantageous for preventing fluid loss from the compartment. Materials from which the device may be made vary. In some non-limiting embodiments, the collection device chamber may be made of the same material as the treatment device chamber.

The device can be designed for use with any wound treatment device as described herein and on any body part, including both human and veterinary applications. Various geometries such as spherical, cubic, parallelepiped, tubular, pouch, envelope or other shapes may be implemented based on the intended application.

In some embodiments, the wound fluid collection device may comprise an absorbant insert 320. The absorbant insert 320 may be made of a substrate, such as an absorbant or a superabsorbent material. The absorbant material may be in sheet, powdered, granular, or other form in the dry state. The absorbant material may be, for example, any hydrophilic polymer, or any porous, fibrous, synthetic, or non-synthetic material. The absorbant material may comprise a gel or polymer. For example, the absorbant material may be a synthetic or a naturally occurring polymer. The absorbant material may generally expand as liquid is absorbed. The absorbant material may gelate with wound material. In some embodiments, the walls of the wound fluid collection chamber 310 may expand as liquid is absorbed. The absorbant insert 320 may prevent collapse of the container when a negative pressure is applied. In some embodiments, the container may be constructed of the same impermeable material that constructs the superstructure of the wound treatment device. This material may be embossed so that the embossed portions face inward into the container and thus provide pathways for airflow in order to enable or facilitate airflow through the fluid trap. In at least some non-limiting embodiments, the absorbant insert may uptake at least 500g of wound material.

The collection device 300 may include a first tube 330 in fluid communication with the chamber of a wound treatment device. The collection device 300 may further include a second tube 340 in fluid communication with a source of suction. In at least some embodiments described herein, a source of suction is capable of generating and/or maintaining sub-atmospheric pressure within an enclosed space. The device may include an inline pump. A one-way valve between the wound treatment device and the fluid collection device may prevent back leakage to the wound treatment device. A safety device, such as a transducer, may be included on the pump side so that airflow may be terminated automatically once the absorbant material is fully saturated.

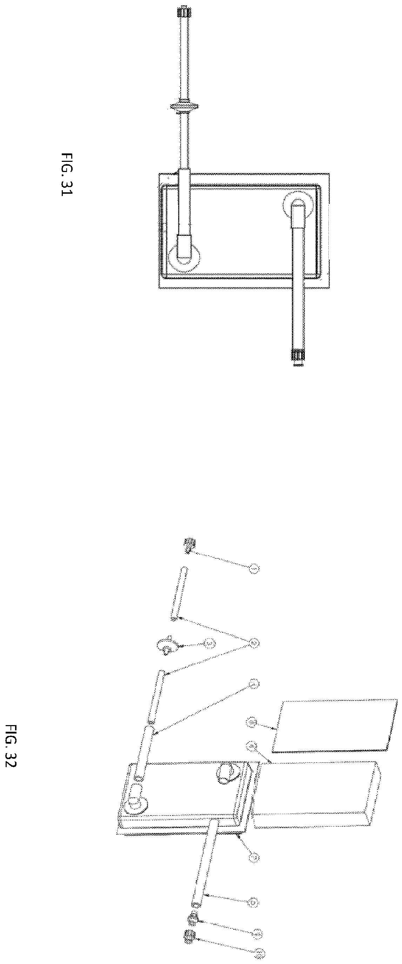

FIG. 31 is a schematic side view of a wound fluid collection device in accordance with one or more embodiments. It is illustrated that a piece of material can be folded and sealed on the remaining three sides around the perimeter to form the collection device. FIG. 32 is an exploded schematic side view of a wound fluid collection device in accordance with one or more embodiments. FIG. 32 illustrates that compartment 7 may include both a cotton material as well as a laminate absorbant sheet. Check valve (one-way) 10 prevents back flow to the wound treatment device and transducer 3 serves as a safety measure as described above.

In some embodiments, the wound fluid collection device is disposable and complies with industry waste material disposal regulations. In some embodiments of the disclosure, a patient is not restricted to a source of electricity or a battery pack. The collection device can be used with ease, so that the patient's mobility is not otherwise compromised. Further, the suction pump preferably has built-in safety limitations on force of suction, duration of operation, and overfilling of the wound fluid collection device.

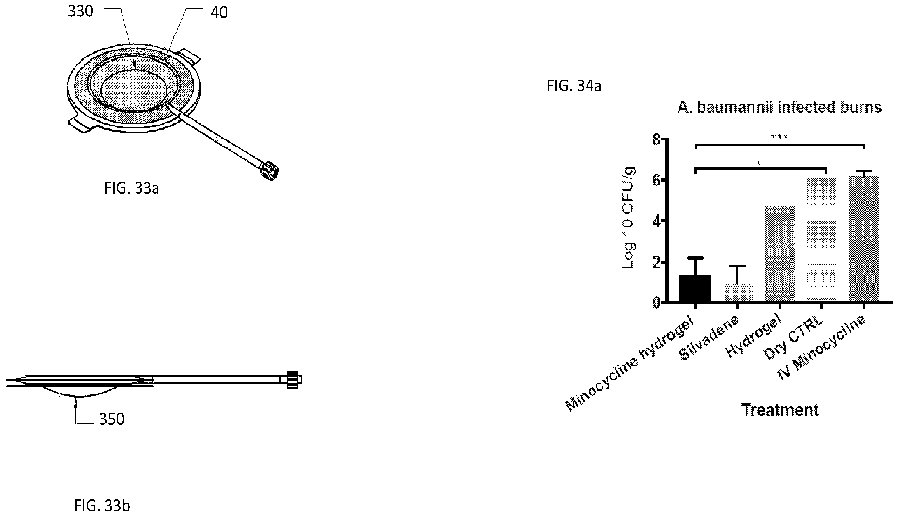

In some embodiments, the wound treatment device chamber may generally involve a degree of convexity or concavity to enable it to be pulled down, for example, into contact with a deep or full thickness wound, such as upon application of negative pressure wound therapy. FIG. 33a illustrates the concavity of a wound treatment device chamber. The wound treatment device chamber, concave with respect to the device as applied, has a wound perimeter area 330, which is placed over the perimeter of the wound. Engineered structures 40 interface with the wound surface during use of the device. The engineered structures may directly contact the wound surface. The concavity of the wound treatment device chamber may be desirable in certain cases such as deep wounds. In a deep wound, the underside of the device may be pulled into the deep parts of the wound, in order to provide negative pressure wound therapy and micromechanical forces to the entire deep wound surface. As shown in FIG. 33b, a wound treatment device chamber has a concave superstructure 350. The concavity of the superstructure of the device may provide folds that extend into the deep part of the wound to create channels for air and fluid flow.

In accordance with one or more embodiments, a kit for wound treatment may include a wound treatment device, formulated therapeutic agent, and/or a wound fluid collection device as described herein. The kit may further include instructions to use one or more of the components for wound treatment.

The function and advantage of these and other embodiments of the materials and methods disclosed herein will be more fully understood from the examples below. The following examples are intended to illustrate the benefits of the disclosed materials and methods, but do not exemplify the full scope thereof.

### EXAMPLE

A swine study was conducted. Up to 14 partial-thickness burns were created on the dorsum of each pig. Subsequently, the burn wounds were infected with S. aureus, P. aeruginosa, A. baumannii or C. albicans and then treated individually for 7 days with 1000xMIC of topical vancomycin, gentamicin, minocycline or 10xMIC of topical diflucan, respectively, formulated in a 0.625% alginate hydrogel. A wound treatment device as described herein was used in conjunction with the antibiotic treatment. Silver sulfadiazine cream (Silvadene), blank 0.625 % alginate hydrogel and IV antibiotics were used as controls for each topical antibiotic hydrogel. On day 7, immediately after euthanasia 6 mm wound tissue punch biopsies were collected from each wound and flash frozen for quantitative bacteriology analysis.

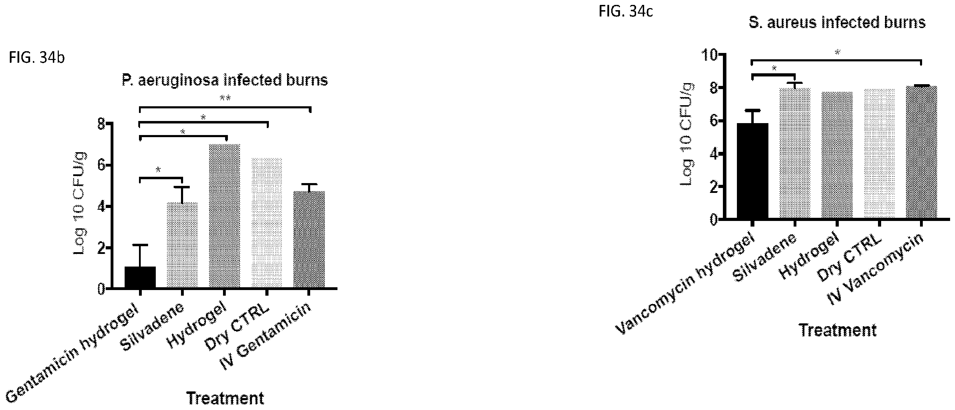

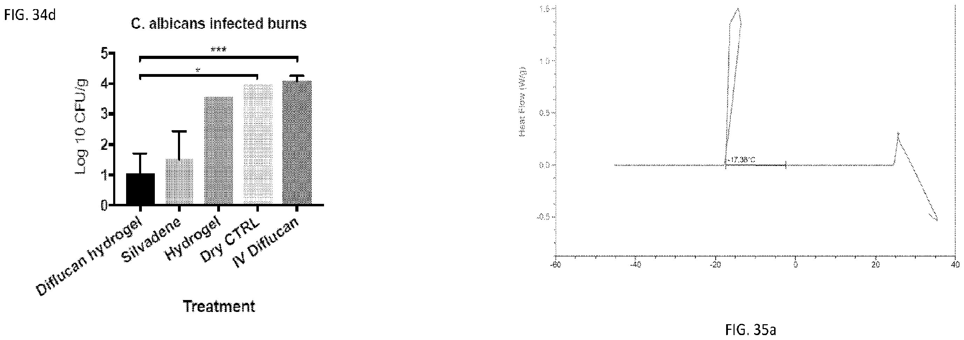

According to FIG. 34a, the results from the burns infected with A. baumannii showed that topical treatment with 1000xMIC minocycline hydrogel reduced bacterial counts more efficiently than IV minocycline and dry control (p<0.001 and p<0.01 respectively). According to FIG. 34b, in P. aeruginosa infected burns 1000xMIC gentamicin hydrogel reduced bacterial counts more efficiently than Silvadine cream (p<0.01), blank hydrogel (p<0.01), dry control (p<0.01) and IV minocycline. According to FIG. 34c, in S. aureus infected wounds, 1000xMIC minocycline hydrogel reduced bacterial counts in the burnt tissue more efficiently than Silvadine cream (p<0.01) and IV minocycline (p<0.01). According to FIG. 34d, in C. albicans infected wounds 10xMlC diflucan hydrogel reduced bacterial counts in the burnt tissue more efficiently than dry control (p<0.01) and IV diflucan (p<0.01).

Thermal stability data pertaining to the 0.625 % alginate hydrogel is presented in FIG. 35a illustrating Differential Scanning Calorimetry (DSC) measurements of the hydrogel sample from room temperature to -45_{°}C at a rate of 0.1_{°}C per minute. The freezing point is indicated by a positive heat flow into the hydrogel sample during the cooling cycle beginning at - 17.38_{°}C.

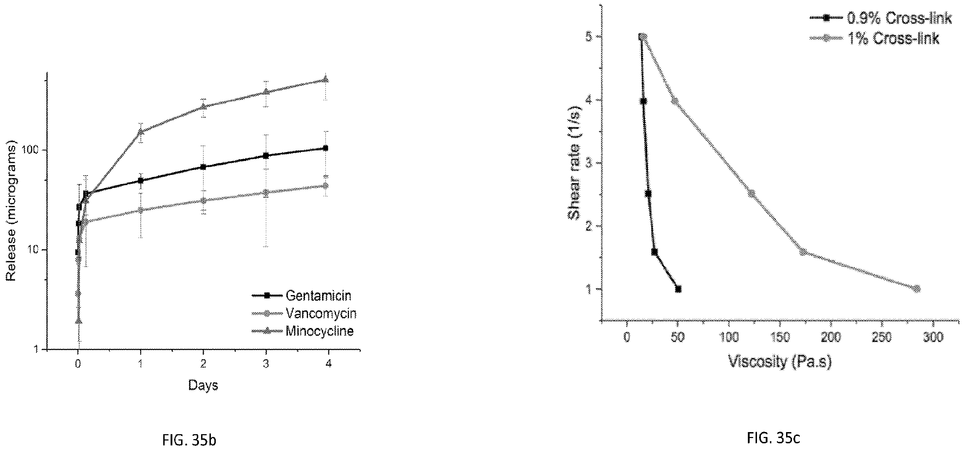

Cumulative drug release data pertaining to the 0.625 % alginate hydrogel is presented in FIG. 35b. Hydrogel samples of 500 microliters were placed in Trans-well membrane plates, with 500 microliters of PBS in the bottom well. At each time point, 100 microliter aliquots were taken from the bottom well, while 100 microliters of fresh PBS was added simultaneously. Aliquots were run on Liquid chromatography-mass spectrometry (LCMS) to quantify the concentrations of the respective antibiotics.

Rheology data pertaining to the 0.625 % alginate hydrogel is presented in FIG. 35c. On a stress-controlled rheometer, homogenized hydrogels were analyzed via shear rate sweeps to calculate the viscosity, as an indication of injectability. The geometry used was rough-surfaced 1_{°} 40mm Cone-and-Plate at a gap of 400 microns.

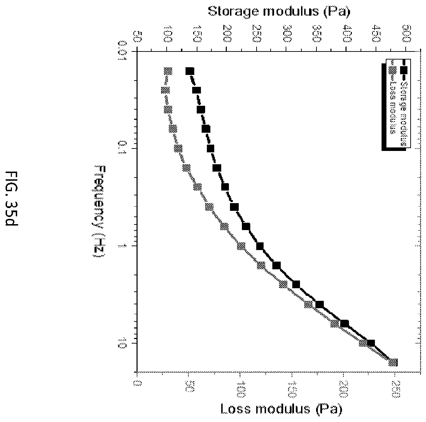

Storage modulus and loss modulus data pertaining to the 0.625 % alginate hydrogel is presented in FIG. 35d. The storage modulus is larger than the loss modulus, indicative of a gel, and not a viscous liquid. Also, the moduli are relatively low, demonstrating that it is a very soft hydrogel. A strain sweep is used to determine the linear-viscoelastic area of the hydrogel, and a frequency sweep is used to determine the linear equilibrium modulus plateau of the hydrogel.

### PROPHETIC EXAMPLE

The goal of this study will be to determine the efficacy of using a wound treatment device in conjunction with formulated high-dose therapeutic agents as described herein to ease pain and reduce or eradicate infection as compared to the standard of care dressing in human patients.

Pre-clinical experiments in swine have demonstrated that immediate treatment with the present wound treatment devices stop wound depth progression and prevent infection.

In this study, a large but safe dose (1000 x MIC) of an antibiotic and analgesic in a gel will be used to immediately treat patients with traumatic extremity wounds and burns (total body surface area of 5 to 30%). There will be 25 standard of care (control) devices and 25 wound treatment devices in accordance with one or more present embodiments used in the study. The devices will be left in place for at least 48 and up to 96 hours at which time it will be removed and the wound cultured and debrided. The antibiotic and analgesic concentrations in the gel/fluid within the devices as well in the serum will be measured every 24 hours.

Quantitative bacterial counts (CFU) will demonstrate significant reduction or eradication of infection in connection with the devices and formulations described herein in comparison to the standard of care. Likewise, pain as assessed by tonometry and a pain analog scale will demonstrate reduction or even elimination of pain compared to the standard of care.

It is to be appreciated that embodiments of the methods, devices, and apparatuses discussed herein are not limited in application to the details of construction and the arrangement of components set forth in the above description or illustrated in the accompanying drawings. The methods, devices, and apparatuses are capable of implementation in other embodiments and of being practiced or of being carried out in various ways. Examples of specific implementations are provided herein for illustrative purposes only and are not intended to be limiting. In particular, acts, elements and features discussed in connection with any one or more embodiments are not intended to be excluded from a similar role in any other embodiment.

Having described above several aspects of at least one embodiment, it is to be appreciated that various alterations, modifications, and improvements will readily occur to those skilled in the art. Such alterations, modifications, and improvements are intended to be part of this disclosure and are intended to be within the scope of the invention to the extent defined by the claims. Accordingly, the foregoing description and drawings are by way of example only.

## Claims

1. A kit for wound treatment, comprising:
a wound treatment device comprising:
a chamber that includes an inner surface and a sealing portion that defines an isolated treatment space;
a plurality of embossed structures arranged in a pattern on the inner surface of the chamber, the structures configured to directly contact a wound and to create pathways for distributing negative pressure between the inner surface of the chamber and the wound; and
at least one tube having a first end connected to the chamber, the at least one tube being in fluid communication with the isolated treatment space so as to enable at least one selected from the group of: applying negative pressure to the isolated treatment space and applying a therapeutic agent to the wound; and
a therapeutic agent comprising an antibiotic formulated at a concentration of up to or at least about 1000 x MIC with a gel.

2. The kit of claim 1, wherein the therapeutic agent further comprises an analgesic.

3. The kit of claim 1, wherein the therapeutic agent formulation includes saline.

4. The kit of claim 1, wherein the structures have a height of about 0.2 mm to about 5mm and spaced about 0.2mm to about 10mm from one another,

5. The kit of claim 1, wherein the structures intrude into the isolated treatment space in a direction generally perpendicular to the inner surface of the chamber.

6. The kit of claim 1, wherein each embossed structure has a shape selected from the group consisting of a cone, a pyramid, a pentagon, a hexagon, a half sphere, a dome, a rod, an elongated ridge with rounded sides, and an elongated ridge with square sides.

7. The kit of claim 1, wherein the chamber is made of a substantially impermeable and/or transparent material.

8. The kit of claim 1, wherein the at least one tube is further configured to remove wound fluid from the treatment space;

9. The kit of claim 8, further comprising a wound fluid collection device housing an absorbant material fluidly connected to the treatment space via the at least one tube.

10. The kit of claim 8, wherein the collection device includes at least one of: an inline pump, a check valve, and a safety transducer.

11. The kit of claim 1, further comprising a source of negative pressure in communication with the chamber via the at least one tube.

12. The kit of claim 1, further comprising instructions for use of the device and the therapeutic agent for wound treatment.

13. The kit of claim 1, wherein the gel is a hydrogel.

## Patentansprüche

1. Kit zur Wundbehandlung, umfassend:
eine Wundbehandlungsvorrichtung, umfassend:
eine Kammer, die eine innere Oberfläche und einen abdichtenden Abschnitt enthält, der einen isolierten Behandlungsraum definiert;
eine Vielzahl von geprägten Strukturen, die in einem Muster auf der Innenfläche der Kammer angeordnet sind, wobei die Strukturen dafür ausgelegt sind, eine Wunde direkt zu berühren und Pfade zur Verteilung von Unterdruck zwischen der Innenfläche der Kammer und der Wunde zu schaffen; und
mindestens ein Rohr mit einem ersten Ende, das mit der Kammer verbunden ist, wobei das mindestens eine Rohr in Fluidverbindung mit dem isolierten Behandlungsraum steht, um mindestens eines ausgewählt aus der Gruppe der Folgenden zu ermöglichen: Aufbringen von Unterdruck auf den isolierten Behandlungsraum und Aufbringen eines therapeutischen Mittels auf die Wunde; und
ein therapeutisches Mittel, das ein Antibiotikum umfasst, das in einer Konzentration von bis zu oder mindestens etwa 1000 x MIC mit einem Gel formuliert ist.

2. Kit nach Anspruch 1, wobei das therapeutische Mittel ferner ein Analgetikum umfasst.

3. Kit nach Anspruch 1, wobei die Formulierung des therapeutischen Mittels eine Kochsalzlösung enthält.

4. Kit nach Anspruch 1, wobei die Strukturen eine Höhe von etwa 0,2 mm bis etwa 5 mm aufweisen und etwa 0,2 mm bis etwa 10 mm voneinander beabstandet sind,

5. Kit nach Anspruch 1, wobei die Strukturen in den isolierten Behandlungsraum in einer Richtung eindringen, die im Allgemeinen senkrecht zur Innenfläche der Kammer verläuft.

6. Kit nach Anspruch 1, wobei jede geprägte Struktur eine Form hat, die aus der Gruppe ausgewählt ist, die aus einem Kegel, einer Pyramide, einem Fünfeck, einem Sechseck, einer Halbkugel, einer Kuppel, einem Stab, einem länglichen Steg mit abgerundeten Seiten und einem länglichen Steg mit quadratischen Seiten besteht.

7. Kit nach Anspruch 1, wobei die Kammer aus einem im Wesentlichen undurchlässigen und/oder transparenten Material hergestellt ist.

8. Kit nach Anspruch 1, wobei der mindestens eine Schlauch dafür konfiguriert ist, Wundflüssigkeit aus dem Behandlungsraum zu entfernen;

9. Kit nach Anspruch 8, das ferner eine Vorrichtung zum Auffangen von Wundflüssigkeit umfasst, die ein absorbierendes Material enthält, das über den mindestens einen Schlauch mit dem Behandlungsraum verbunden ist.

10. Kit nach Anspruch 8, wobei die Sammelvorrichtung mindestens eines der Folgenden enthält: eine Inline-Pumpe, ein Rückschlagventil und einen Sicherheitswandler.

11. Kit nach Anspruch 1, das außerdem eine Unterdruckquelle umfasst, die über den mindestens einen Schlauch mit der Kammer verbunden ist.

12. Kit nach Anspruch 1, das ferner eine Gebrauchsanweisung für die Vorrichtung und das therapeutische Mittel zur Wundbehandlung umfasst.

13. Kit nach Anspruch 1, wobei das Gel ein Hydrogel ist.

## Revendications

1. Trousse pour traitement de blessure, comprenant :
un dispositif de traitement de blessure comprenant :
une chambre qui inclut une surface intérieure et une partie d'étanchéité qui définit un espace de traitement isolé ;
une pluralité de structures gaufrées agencées en un motif sur la surface intérieure de la chambre, les structures étant configurées pour entrer directement en contact avec une blessure et pour créer des voies pour distribuer une pression négative entre la surface intérieure de la chambre et la blessure ; et
au moins un tube ayant une première extrémité raccordée à la chambre, l'au moins un tube étant en communication fluidique avec l'espace de traitement isolé afin de permettre au moins un sélectionné parmi le groupe de : l'application de pression négative sur l'espace de traitement isolé et l'application d'un agent thérapeutique sur la blessure ; et
un agent thérapeutique comprenant un antibiotique préparé à une concentration pouvant atteindre ou d'au moins environ 1000 x MIC avec un gel.

2. Trousse selon la revendication 1, dans laquelle l'agent thérapeutique comprend en outre un analgésique.

3. Trousse selon la revendication 1, dans laquelle la préparation d'agent thérapeutique inclut une solution saline.

4. Trousse selon la revendication 1, dans laquelle les structures ont une hauteur d'environ 0,2 mm à environ 5 mm et sont espacées d'environ 0,2 mm à environ 10 mm les unes des autres.

5. Trousse selon la revendication 1, dans laquelle les structures pénètrent dans l'espace de traitement isolé dans une direction généralement perpendiculaire à la surface intérieure de la chambre.

6. Trousse selon la revendication 1, dans laquelle chaque structure gaufrée a une forme sélectionnée parmi le groupe constitué de : un cône, une pyramide, un pentagone, un hexagone, une demi-sphère, un dôme, une tige, une saillie allongée avec des côtés arrondis, et une saillie allongée avec des côtés carrés.

7. Trousse selon la revendication 1, dans laquelle la chambre est faite d'un matériau sensiblement imperméable et/ou transparent.

8. Trousse selon la revendication 1, dans laquelle l'au moins un tube est en outre configuré pour éliminer un fluide de blessure à partir de l'espace de traitement.

9. Trousse selon la revendication 8, comprenant en outre un dispositif de collecte de fluide de blessure logeant un matériau absorbant fluidiquement raccordé à l'espace de traitement par l'intermédiaire de l'au moins un tube.

10. Trousse selon la revendication 8, dans laquelle le dispositif de collecte inclut au moins un de : une pompe en ligne, un clapet anti-retour, et un transducteur de sécurité.

11. Trousse selon la revendication 1, comprenant en outre une source de pression négative en communication avec la chambre par l'intermédiaire de l'au moins un tube.

12. Trousse selon la revendication 1, comprenant en outre des instructions pour l'utilisation du dispositif et de l'agent thérapeutique pour traitement de blessure.

13. Trousse selon la revendication 1, dans laquelle le gel est un hydrogel.
